(19) Europäisches Patentamt
European Patent Office
Office européen des brevets

(11) EP 3 634 366 B1

(12) EUROPEAN PATENT SPECIFICATION

(45) Date of publication and mention of the grant of the patent:
**25.11.2020 Bulletin 2020/48**

(21) Application number: **18726984.0**

(22) Date of filing: **23.05.2018**

(51) Int Cl.:
***A61K 8/34*** *(2006.01)* ***A61K 8/39*** *(2006.01)*
***A61K 8/49*** *(2006.01)* ***A61Q 11/02*** *(2006.01)*
***A61K 8/73*** *(2006.01)* ***A61K 8/81*** *(2006.01)*
***A61K 8/04*** *(2006.01)* ***A61K 8/20*** *(2006.01)*
***A61K 8/22*** *(2006.01)*

(86) International application number:
**PCT/EP2018/063514**

(87) International publication number:
**WO 2018/224311 (13.12.2018 Gazette 2018/50)**

(54) **A COMPOSITION, METHOD AND KIT FOR WHITENING TEETH**

ZUSAMMENSETZUNG, VERFAHREN UND KIT ZUM BLEICHEN VON ZÄHNEN

COMPOSITION, PROCÉDÉ ET KIT DE BLANCHIMENT DES DENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.06.2017 PCT/CN2017/087314**
**20.06.2017 EP 17176862**

(43) Date of publication of application:
**15.04.2020 Bulletin 2020/16**

(73) Proprietors:
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
- **Unilever PLC**
**London, Greater London EC4Y 0DY (GB)**
Designated Contracting States:
**CY GB IE MT**

(72) Inventors:
- **GHATLIA, Naresh, Dhirajlal**
**Whitefield**
**Bangalore 560 066 (IN)**
- **LAHORKAR, Praful, Gulab, Rao**
**Whitefield**
**Bangalore 560 066 (IN)**
- **VAIDYA, Ashish, Anant**
**Whitefield**
**Bangalore 560 066 (IN)**

(74) Representative: **Tansley, Sally Elizabeth**
**Unilever N.V.**
**Unilever Patent Group**
**Bronland 14**
**6708 WH Wageningen (NL)**

(56) References cited:
**EP-A1- 3 150 226** **EP-A2- 1 951 184**
**WO-A2-2014/042936** **US-A- 6 149 895**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 634 366 B1

## Description

### Field of the Invention

The present invention relates to oral care compositions for whitening teeth. More specifically, the invention relates to such composition and a kit that contain a gel having a photosensitizer.

### Background of the Invention

The colour of our teeth is influenced by a combination of their intrinsic colour and the presence of any extrinsic stains that may form thereon. Staining is linked to the adsorption of materials into the pellicle on the surface of enamel. Factors that influence extrinsic stain formation include poor brushing techniques, smoking, dietary intake of coloured foods (e.g. red wine), age and the use of certain cationic agents such as chlorhexidine or metallic salts of tin and iron. Consumers have strong desire for whiter teeth and some individuals are dissatisfied with the inherent colour of their teeth or with the stains. This desire for whiter teeth has given rise to a growing trend of whitening products which range from toothpastes to mouthwashes and chewing gums.

Toothpastes meant for whitening rely on an optimization of abrasive and chemical components for removal of stains. During brushing, these abrasive particles get temporarily trapped between the toothbrush and the stained surface (of teeth) to abrade away the stains. Chemical components may also be used, usually in conjunction with abrasive particles, and these include calcium chelators, polymers, surfactants, enzymes and oxidising agents. EP 1935395 A1 (Unilever) describes a novel optical approach to tooth whitening in which the toothpaste contains a blue pigment (in particular Blue Covarine) which gets deposited on the surface of teeth. Here it is able to alter the optical effects of surface which leads to a perception of whiter teeth. Deposition is aided by GANTREZ® type of polymers.

One of the more prevalent methods involves the use of dentifrices which contain a bleaching agent such as hydrogen peroxide with one or more other bleaching agents. Exemplary of such disclosures are US2002122776 A and WO0018366 A1 (Both Unilever).

US2003194383 AA (Unilever) discloses a kit and method for whitening teeth which utilizes a U-shaped dental tray fittable over a person's teeth and a bleaching composition formed from a combination of first and second compositions, which prior to use, are stored separately from one another but need to be mixed soon before use. The first composition includes a peroxide and the second composition includes an alkaline material.

It is known to photobleach stained teeth by applying a photobleaching composition containing a photosensitizer (chromophores/dyes), followed by exposure to light such as UV, visible, LED light. Sometimes an applicator (tray) is used to facilitate the application and contact with teeth. Usually such compositions contain a bleach/peroxide, or the compositions are meant to be pre-mixed with a bleach before application.

In the past people used photoactivatable compositions containing per-compounds. In this case, a user needs to first apply the composition (usually a gel) containing the percompound. Thereafter (s)he needs to activate the percompound by exposing it (i.e. the composition) to radiation which may be UV or visible. The light decomposes the percompound quickly to release free radicals which act against the stains to bleach them.

Such compositions are effective when applied for shorter periods. Accordingly, sensitivity due to, e.g., percolation of hydrogen peroxide to the pulp tissue (e.g., pulpal inflammation), may be minimized or eliminated.

A more recent trend is to use the compositions which contain a percompound and a photoactivatable ingredient which is usually a dye.

An advantage of such compositions is that they do not require compounds that generate heat, and therefore, discomfort associated therewith may be eliminated. Usually dyes (e.g., Eosin) are used as the photoactivatable dyes. Such dyes are used in conjunction with the one or more bleaching agents. The dyes have emission wavelength e.g. from about 400 to 570 nm.

WO2011084746 A1 and WO11084744 A1 (Both Colgate) discloses the use of GRAS (Generally Regarded As Safe) dyes as photosensitizing dyes in oral compositions for anti-bacterial and anti-inflammatory efficacy. The compositions provide strong anti-bacterial activity upon irradiation with absorbable, visible light. The anti-bacterial activity is administered in about two minutes. The GRAS dyes per se exhibit little or no anti-bacterial activity. However, their anti-bacterial properties are turned on when the compositions are exposed to absorbable, visible light. The compositions contain about 70 wt% organic solvents.

US2009238778 AA (MCNEIL PPC) discloses tooth whitening compositions that are capable of being activated by light energy. The compositions contain riboflavin or a derivative of riboflavin, a bleaching agent and a water-soluble liquid phase.

US80260660 A1 (S & C POLYMER SILICON U COMPOSITE SPEZIALITATEN GMBH) discloses dental bleaching compositions comprising riboflavin and/or riboflavin derivatives that through the use of radiation energy provide for more rapid decomposition of oxidising agent contained in the bleaching agent and thereby effectively accelerate the

bleaching action. The gel compositions disclosed in this patent application contain very high amount of organic solvent(s) and the compositions also contain per-compounds for bleaching action.

EP2338465 A1 (Klox Technologies Inc, 2011) discloses faster acting compositions with minimal or no sensitivity post the treatment. The compositions contain oxidizing agent (e.g., a peroxide), and an activating agent having emission wavelength of 400 to 570 nm (e.g., Eosin B).

US2006141422A (French Transit LLC) discloses compositions which are effective against stains at lower levels of active oxidizing agents and lower levels of heat than known devices. The compositions contain per-compounds and organic solvents.

US2014105832 AA (Valent Pharmaceuticals Int Inc) discloses light-activated teeth whitening compositions (gels) which contain an oxidizing agent and an activating agent that has an emission wavelength between about 400 nm and about 570 nm. The activating agent is a combination of Eosin Y and Fluorescein. The compositions contain substantial amount of organic solvents. The compositions are formulated to minimize post-treatment sensitivity.

WO9940870 A1 (Discus Dental LLC) discloses a kit and a method of de-staining teeth using a hand-held source of actinic radiation. The compositions which are disclosed in this application contain substantial amount of peroxide.

The efficacy of such compositions and kits can be measured and assessed by a term known as Whiteness Index Observed (WIO).

Luo et al. proposed a modified version of the CIE whiteness index in Garcia JA, Rubino M, Romero J, Hita E. Measuring The Whiteness Of Human Teeth. Color Research and Application 1993; 18: 349 - 52. It is based directly on observers scoring of the Vita® 3D Shade Guide and is defined as:

$$WIO = Y + 1075.012\ (x_p - x) + 145.516\ (y_p - y)$$

We have surprisingly determined that some hitherto unreported formulation changes in the teeth whitening gel composition leads to incremental increase in Whiteness Index of hydroxyapatite plates (often used as substitutes of teeth in experiments) with each successive treatment, even with an intervening step of brushing (using a toothpaste) between two successive treatments. What is even more surprising is that the effect is observed in the absence of oxidising agents.

## Summary of the Invention

It is believed that synergy of a selective gelling agent with a particular type of photosensitizer increases the WIO. Such an effect is not observed with at least some of the usual gelling agents such as xanthan gum, which are generally used in such compositions.

In accordance with a first aspect is disclosed an aqueous gel composition for whitening teeth, comprising:

(i) at least one photosensitizer which is selected from a xanthene class of dye;
(ii) maximum of 10 wt% organic solvents;
(iii) less than 0.1 wt% oxidising agent; and,
(iv) a polymeric gelling agent other than those containing galactose, galactomannan or glucosamine units in their chemical structure.

In accordance with a second aspect is disclosed a kit-of-parts comprising:

(i) an aqueous gel composition according to the first aspect;
(ii) a source of actinic light;
(iii) optionally, an applicator for applying said composition to teeth; and,
(iv) further optionally, a set of instructions for use of said kit-of-parts.

In accordance with a third aspect is disclosed a device for whitening teeth comprising:

(i) an aqueous gel composition as claimed in any of claims 1 to 8
(ii) an applicator configured to apply to the teeth said aqueous gel composition; and,
(iii) a source of actinic light integral with said device.

In accordance with a fourth aspect is disclosed a method of whitening teeth comprising:

(i) providing an aqueous gel composition according to the first aspect;

(ii) applying said gel to teeth; and,
(iii) exposing said gel, so applied, to a source of actinic light.

In accordance with a fifth aspect is disclosed the use of an aqueous gel composition according to the first aspect for whitening teeth.
In accordance with a sixth aspect is disclosed a packaged product comprising an aqueous gel composition of the first aspect.
Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.
As per this invention the term 'A derivative of xanthene' is meant one or more dye selected from the 'Xanthene class of dyes'. The two terms are used interchangeably.
All references to the terms wt% or % by weight shall be construed as referring to the % of the concerned ingredient by weight of the composition, unless indicated to the contrary.

## Detailed Description of the Invention

The term "actinic light" is intended to mean light energy emitted from a specific light source (e.g. visible, UV, lamp, LED, or laser) and capable of being absorbed by matter (e.g. the chromophore or photoactivator defined below). It is preferred that the actinic light is visible light.
The term "photosensitizer" refers to any agent capable of absorbing the actinic light. The photosensitive agent undergoes photoexcitation and transfers its energy. This may be in the form of emitted light (e.g. fluorescence) and/or energy transferred to molecules. This energy may initiate chemical reactions. Photosensitizers may enhance and/or accelerate the dispersion of light energy, or otherwise enhance and/or activate the decomposition of an oxidizing agent.
The term "chromophore" means a chemical compound or a part of a chemical compound, which when contacted by light irradiation, is capable of absorbing the light. The chromophore readily undergoes photoexcitation and then emits energy such as by transferring its energy to other molecules or by emitting light.
When referring to the pH of the aqueous gel composition, the pH is measured when 1 part by weight of the composition is uniformly dispersed and/or dissolved in 20 parts by weight pure water at 25 °C. In particular, the pH may be measured by manually mixing 1 g composition with 20 ml water for 30 seconds, then immediately testing the pH with indicator paper or a pH meter.
"Substantially free of, as used herein, means comprising less than 1.5%, and preferably less than 1.0%, and more preferably less than 0.75% and more preferably still less than 0.5%, and even more preferably less than 0.1 % and most preferably from 0.0 to 0.01 % by weight, based on total weight of the aqueous gel composition, including all ranges subsumed therein.
Viscosity of the aqueous gel composition is the value observed and recorded at room temperature (25 °C) with a Brookfield Viscometer, Spindle No. 4 and at a speed of 5 rpm. Values are quoted in centipoises (cP = mPa.s) unless otherwise specified.
Stain teeth could become troublesome, whether the cause for it is intrinsic or an extrinsic factor or agent like certain beverages chlorhexidine. The accumulation of stains on teeth poses an aesthetic problem for some individuals. Stains are usually extrinsic in nature and generally represent discoloration of the pellicle and/or plaque. The exact mechanisms involved in stain formation may not be fully understood. It is suggested that pigments produced by chromagenic bacteria, colored products from the chemical transformation of pellicle components and adsorption/retention of dietary constituents contribute to extrinsic stains. The dietary factors which appear to contribute heavily towards staining include coffee, tea, and red wines, as well as smoking. In addition to chlorhexidine, staining is promoted by other cationic antimicrobial agents such as hexetidine or quaternary ammonium compounds.
The index proposed by Luo et al (known as WIO) has the same functional form as some of the previous whiteness indices but the parameters are different and are optimized specifically for the evaluation of whiteness in teeth. It is regarded as most appropriate for assessing changes in teeth whiteness.

## The gel composition of the Invention

The gel composition of the invention is an aqueous gel. The term aqueous refers to there being a substantial

amount of water which acts like the continuous medium in which other ingredients are present.

The composition of the invention is meant for whitening teeth which implies that it is useful for maintaining the whiteness of teeth. Alternatively, the composition of the invention is useful for removing or lightening, at least partly, some of the common stains such as tea or coffee stain.

Compositions of the invention include at least one photosensitizer which is a selected from a xanthene class of dye. Alternatively, the compositions of the invention include more than one, i.e., a combination of such photosensitizers.

One of the general structures of xanthene class of dye is as follows:

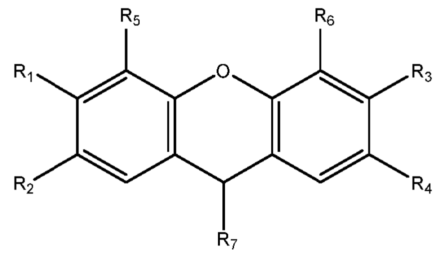

Where R1, R2, R3, R4, R5 and R6 are functional groups such as -OH, H, Br, I, Cl, amines, ketones, $O^-M^+$(where M is metal ion) and R7 is organic moiety / derivative such substituted aromatic or benzofuran.

It is preferred that the derivative of xanthene is at least one of Acid Red 52, Gallein, Acid Red 289, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein (contains Mono-, Tri- and Tetra-), Acid Red 91, Uranine K, Fluorescein, Uranine, Pyrogallol Red, Rhodamine 6G, Rhodamine B, Acid Red 94, Rhodamine 800 Sulfonfluorescein, Tetrabromofluorescein Potassium Salt, Acid Red 87 2',4',5',7'-Tetrabromo-3,4,5,6-tetrachlorofluorescein, Acid Red 92 3,4,5,6-Tetrachlorofluorescein, Tetraiodofluorescein, Phenylfluorone and Erythrosine B, Rose Bengal, Eosin, Phloxine B, Fluorescein or Erythrosine.

As per an especially preferred aspect of the invention the photosensitizer is selected from a xanthene class of dye selected from one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red, Rhodamine 6G, Rhodamine B, Sulfonfluorescein, Tetrabromofluorescein Potassium Salt, Acid Red 91 (Eosin Bluish), Acid Red 94 (Rose Bengal), Acid Red 87(Eosin), Acid Red 92 (Phloxine B), 3,4,5,6-Tetrachlorofluorescein, Tetraiodofluorescein (Erythrosin B or Erythrosine B) and Phenylfluorone

However, it is particularly preferred that the photosensitizer is at least one of Rose Bengal, Eosin, Phloxine B, Fluorescein or Erythrosine.

It is also preferred that the composition of the invention comprises 0.001 to 2 wt% of the photosensitizer. Where a combination of photosensitizers is comprised in the composition, the above mentioned range applies to the total amount thereof. It is further particularly preferred that the composition of the invention comprises 0.001 to 1 wt% of the photosensitizer, further preferably 0.001 to 0.5 wt% of the photosensitizer.

General structure of a preferred form of xanthene dye is shown below:

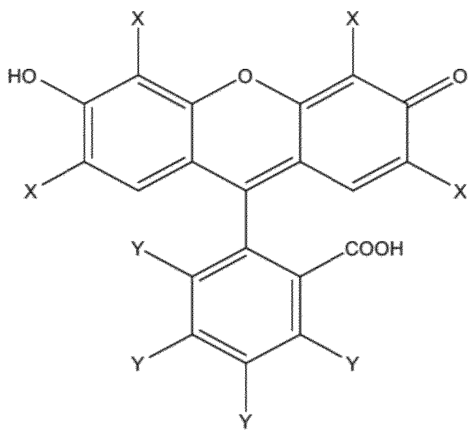

Referring to the chemical structure indicated above, in Fluorescein the X=H and Y=H; in Eosin the X-Br and Y=H; in Erythrosine the X=I and Y=H and in Rose Bengal the X=I and Y=Cl.

The structures of the various type preferred photosensitizers of the xanthene class are:

Acid Red 52

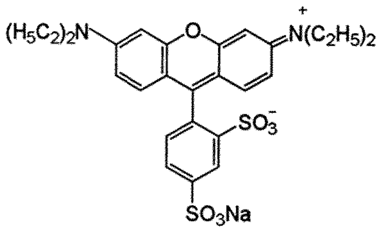

Gallein

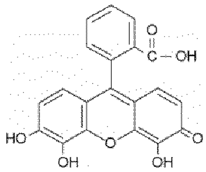

Acid Red 289

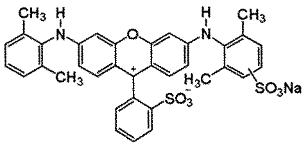

Bromopyrogallol Red

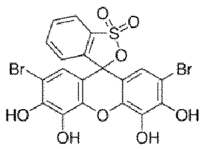

2',7'-Dichlorofluorescein Sodium Salt

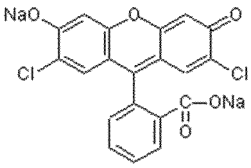

2',7'-Dichlorofluorescein Sodium Salt, 9-(4'-Dimethylaminophenyl)-2,6,7-trihydroxyfluorone Sulfate Hydrate

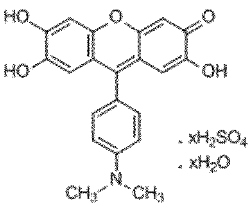

Dibromofluorescein

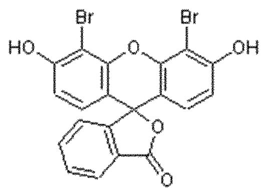

Acid Red 91(Eosin Bluish)

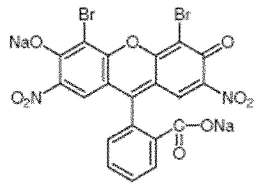

Uranine K

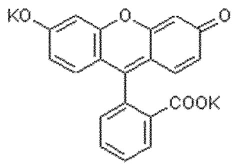

Uranine (Fluorescein Sodium Salt)

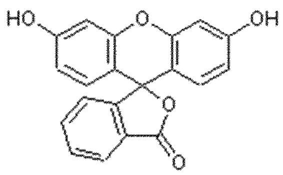

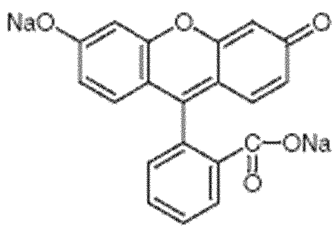

Pyrogallol Red

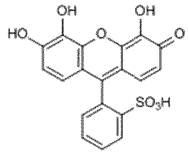

Rhodamine 6G

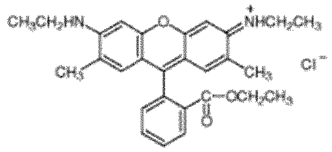

Rhodamine B

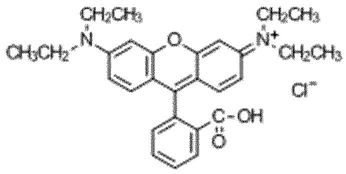

Acid Red 94 (Rose Bengal)

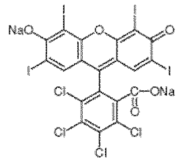

Rhodamine 800

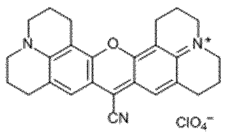

Tetrabromofluorescein Potassium Salt

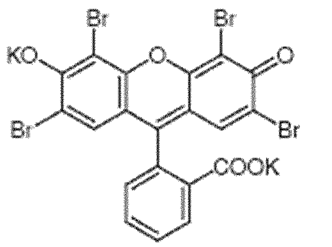

Acid Red 87 (Eosin) (Tetrabromofluorescein Sodium Salt)

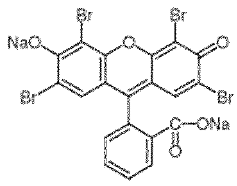

3,4,5,6-Tetrachlorofluorescein

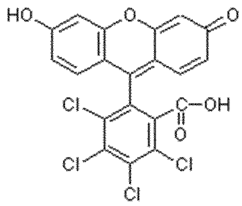

Tetraiodofluorescein(Iodoeosine)- Erythrosin B

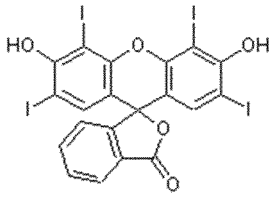

Phenylfluorone

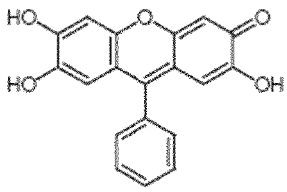

Acid red 92 (Phloxine B)

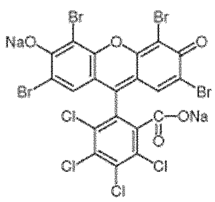

In the case of compositions that contain a bleach, it is believed that photosensitizers, when exposed to actinic light, accelerate the dispersion of light energy which consequently leads to photochemical activation of the oxidizing agent within the composition. However, in the case of the present invention, when the photosensitizer is exposed to actinic light, it produces reactive excited species which in turn interact with the stains on teeth to thereby lighten the stains. The result is whiter teeth.

The compositions of the invention comprise a maximum of 10 wt% of one or more organic solvents. In other words, the presence of organic solvents is optional. However, the compositions of prior art almost always have one more organic solvents, usually in appreciable amounts.

However, whenever present, the compositions of the invention comprise 0.5 to 8 wt% of organic solvent. The amount refers to the amount of the solvent in the aggregate, even if there is more than solvent.

Preferably the organic solvent is at least one of glycerine, sorbitol, propyleneglycol, polyethyleneglycol or ethyleneglycol. We have observed that an excess of such solvents has negative effect on the WIO. The WIO is as its maximum when the amount of the solvent is at its minimum which indicates that there is an inverse proportion between the two variables. Beyond an amount of more than 10% (as exemplified herein at least by glycerine), the WIO decreases sharply.

The compositions of the invention comprise less than 0.1 wt% oxidising agent. Which means when such an agent is present, the amount thereof, in the aggregate, is less than 0.1 wt%. Preferably the amount is less than 0.05 wt% and more preferably the amount is less than 0.025 wt%, even more preferably less than 0.01 wt%.

The oxidising agent is chlorine dioxide or sodium chlorite or a peroxide, a peroxyacid, a percarbonate, a perborate, a persulphate, a perphosphate or a monoperoxyphthalate.

The compositions of the invention comprise a polymeric gelling agent other than those containing galactose, galactomannan or glucosamine units in their chemical structure.

It is preferred that the polymeric gelling agent is at least one of a carbomer, a modified cellulose or a copolymer of ammonium acryloyldimethyltaurate and vinylpyrrolidone, gelatin or polyvinyl alcohol. The term carbomer includes the class of acrylates / R-methacrylate copolymer or crosspolymer; or an acrylate / R-alkyl acrylate crosspolymer, where R stands for an alkyl group.

Preferred carbomers include those commercially available from B. F. Goodrich as the CARBOPOL(R)series, including CARBOPOL(R) 934, 940, 941 and 956.

Other preferred grades include acrylates/C10-30 alkyl acrylate crosspolymers which are commercially available as ULTREZ(R) 21, PEMULEN(R) TR-1, and PEMULEN(R) TR-2, from Noveon Corporation.

Preferred modified celluloses include hydroxyethyl cellulose, sodium carboxymethyl cellulose, methyl cellulose and hydroxypropyl cellulose. Other preferred modified celluloses include ethyl hydroxyethyl cellulose (EHEC), carboxymethylhydroxyethyl cellulose (CMHEC), hydroxypropyl hydroxyethyl cellulose (HPHEC), methylhydroxypropyl cellulose (MHPC), methylhydroxyethyl cellulose (MHEC), carboxymethyl methyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethyl hydroxyethyl cellulose (HMEHEC), hydrophobically modified carboxymethylhydroxyethyl cellulose (HMCMHEC), hydrophobically modified hydroxypropyl hydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC) and cationic hydrophobically modified hydroxyethyl cellulose (cationic HMHEC).

An example of a copolymer of ammonium acryloyldimethyltaurate and vinylpyrrolidone is Aristoflex® AVC. Other suitable polymeric gelling agents of this class include those commercially available, for example from Clariant under the ARISTOFLEX® tradename, such as ARISTOFLEX® AVS (INCI name: sodium acryloyldimethyltaurate/VP Copolymer), ARISTOFLEX® HMB (INCI name: ammonium acryloyldimethyltaurate/Beheneth-25 Methacrylate Crosspolymer) and ARISTOFLEX® TAC (INCI name: ammonium acryloyldimethyltaurate/Carboxyethyl Acrylate Crosspolymer).

The polymeric gelling agents which are not suitable for the compositions of the present invention are those containing galactose, galactomannan or glucosamine units in their chemical structure. Examples include the agents used very commonly such as xanthan gum, guar gum, locust bean gum, carrageenan, acacia gum and chitosan.

Guar gum is polysaccharide composed of the sugars galactose and mannose. Locust bean gum contains galactomannan units. Carrageenans are high-molecular-weight polysaccharides made up of repeating galactose units and chitosan contains glucosamine units. Xanthan gum is a high molecular weight hetero-polysaccharide. Its main chain is made up of glucose units. The side chain is a trisaccharide, consisting of alpha-D-mannose that contains an acetyl group, beta-D-glucuronic acid, and a beta-D-mannose terminal unit, linked to a pyruvate group. Acacia gum is a complex and variable mixture of arabinogalactan oligosaccharides, polysaccharides and glycoproteins. They are mainly formed by chains of 3,6-linked $\beta$-D-Galactopyranose substituted in position 6 by side chains of 3-linked $\alpha$-L-arabinofuranose.

It is preferred that the compositions of the invention comprise 0.001 to 5 wt% of the polymeric gelling agent. This amount refers to the aggregate when more than one gelling agent is present. It is preferred that compositions of the invention comprise 0.001 to 2 wt% of the polymeric gelling agent. More preferably the compositions comprise 0.1 to 1 wt% of the polymeric gelling agent.

It is believed that the polymeric gelling agent exerts positive influence on the L* value which indicates that its presence increases the whiteness of teeth. For users of such compositions, the cumulative or additive effect of such applications also carries a lot of significance therefore compositions that allow for an incremental increase in whiteness cycle-after-cycle of application, are very much preferred by the consumers. We have observed that the presence of the selective gelling agent leads to an incremental increase in whiteness of teeth which cumulatively manifests itself as a significant and noticeable increase in WIO of stained HAP discs which are used as substitutes of teeth in view of their compositional similarity to human teeth.

The composition of the invention is a gel. It is preferred that viscosity of the gel is from 200 to 100,000 cps, more preferably 2000 to 50,000 cps.

In particular, when the polymeric gelling agent is a copolymer of ammonium acryloyldimethyltaurate and vinylpyrrolidone, it is preferred that the viscosity is 2000 to 20000 cps. Further, when the polymeric gelling agent is hydroxyethyl cellulose, it is preferred that the viscosity is 200 to 2000 cps.

In a preferred aspect, the aqueous gel composition of the invention comprises Erythrosine B photosensitizer and a copolymer of ammonium acryloyldimethyltaurate and vinylpyrrolidone as the polymeric gelling agent.

Alternatively, Riboflavin is the photosensitizer and a copolymer of ammonium acryloyldimethyltaurate and vinylpyrrolidone is the polymeric gelling agent.

Further alternatively, Rose Bengal is the photosensitizer and a copolymer of ammonium acryloyldimethyltaurate

and vinylpyrrolidone is the polymeric gelling agent.

Further alternatively, Phloxine B is the photosensitizer and a copolymer of ammonium acryloyldimethyltaurate and vinylpyrrolidone is the polymeric gelling agent.

Further alternatively, Eosin is the photosensitizer and a copolymer of ammonium acryloyldimethyltaurate and vinylpyrrolidone is the polymeric gelling agent.

Further alternatively, Rose Bengal is the photosensitizer and a modified cellulose is the polymeric gelling agent.

Further alternatively, Rose Bengal is the photosensitizer and a carbomer is the polymeric gelling agent.

Further alternatively, Rose Bengal is the photosensitizer and gelatin is the polymeric gelling agent.

Further alternatively, Rose Bengal is the photosensitizer and polyvinylalcohol is the polymeric gelling agent.

The pH of the aqueous gel composition of the invention is preferably from 3 to 11, and more preferably from 6 to 8.

The composition according to the invention may optionally include further ingredients to enhance performance and/or consumer acceptability, which are briefly discussed hereinafter.

Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

The level of surfactant may generally range from 0.2 to 15% by total weight surfactant based on the total weight of the composition.

Also suitable are fluoride sources such as sodium fluoride, stannous fluoride, sodium monofluorophosphate, zinc ammonium fluoride, tin ammonium fluoride, calcium fluoride, cobalt ammonium fluoride and mixtures thereof; plant-derivable antioxidants such as flavonoid, catechin, polyphenol, and tannin compounds and mixtures thereof; antioxidant vitamins such as tocopherols and/or derivatives thereof, ascorbic acid and/or derivatives thereof and mixtures thereof. Further optional ingredients customary in the art such as buffers, flavouring agents, sweetening agents, colouring agents, opacifying agents and preservatives.

## The packaged product of the Invention

In accordance with another aspect is disclosed a packaged product comprising the aqueous gel composition of the invention. The gel composition is preferably packaged in a collapsible tube such that it becomes easy to dispense a small amount without much dexterity. Alternatively, the composition is packaged in another type of container, such as a bottle or a jar, preferably with suitable means for dispensing a known quantity of the composition. The pack may be further packed in a suitable secondary package like a carton or a box, preferably with useful information about the contents and instructions for use.

## The kit-of-parts of the Invention

In accordance with a further aspect, the invention relates to a kit-of-parts comprising:

(i) an aqueous gel composition of the first aspect;
(ii) a source of actinic light;
(iii) optionally, an applicator for applying said composition to teeth; and,
(iv) further optionally, a set of instructions for use of said kit-of-parts.

The composition in accordance with the invention needs to be used in conjunction with a suitable source of actinic light.

Any suitable source of actinic light can be used so long as the light is able to activate the photosensitizer belonging to xanthene group. The primary characteristic of suitable sources of actinic light will be that they emit light in a wavelength (or wavelengths) appropriate for photosensitization of the photosensitizer present in the composition. Preferably the source of actinic light is halogen bulb, LED or plasma arc lamp, or laser such as an argon laser, potassium-titanyl phosphate (KTP) laser, LED photocuring device. The radiation is preferably of the visible range but it may be UV or IR. It is preferred that the actinic light has a wavelength in the range from about 400 nm to about 700 nm. In another embodiment, the actinic light has wavelength in the range from about 420 nm to about 560 nm, more preferably 435 nm to about 550 nm.

The applicator is preferably the one which is disclosed in US 20030194383 A (Unilever). This applicator or tray comprises a U-shaped dental tray fittable over a person's teeth which permits contact of the composition of the invention with all possible surfaces surrounding the teeth. Alternatively, the applicator is any other suitable applicator known in the art.
The set of instructions is preferably in the form of a pamphlet containing useful information about the contents and step-by-step instructions for use of the compositions of the invention.

The device of the Invention

In accordance with another aspect is disclosed a device for whitening teeth comprising:

(i) an aqueous gel composition as claimed in any of claims 1 to 8
(ii) an applicator configured to apply to the teeth said aqueous gel composition; and,
(iii) a source of actinic light integral with said device.

As an example, reference may be made to the fittable tray disclosed earlier, with provision for flashing actinic light. In such a case it is preferred that the device comprises a switch or button to switch on and off the light source. The device preferably also includes a battery for supplying energy to the source of light. In such a case it is preferred that the device is easy to use.

The method of the Invention

In accordance with another aspect is disclosed a method of whitening teeth comprising:

(i) providing an aqueous gel composition of the first aspect;
(ii) applying said gel to teeth; and,
(iii) exposing said gel, so applied, to a source of actinic light.

Preferably the gel is applied using the applicator as disclosed earlier. After application, the teeth are exposed to actinic light preferably for 5 to 45 minutes, more preferably for less than 10 minutes. Alternatively, the exposure lasts less than 5 minutes, such as 4, 3, 2, or 1 minute. The application may be repeated as often as desired, e.g. 2 to 3 times a day and 3 to 4 days in a fortnight. The method preferably results in at least one unit of shade difference on the Vita® Shade card, preferably at least 2 units and more preferably at least 3 units. The method is relatively free of post-treatment sensitivity. In some other cases, there is minimal or acceptable level of post-treatment sensitivity. The whitening effect may diminish over time following a treatment. In that case, repeat of the treatment is advisable.
The method for whitening the teeth may be performed in a dentist's office or clinic under ordinary or supervised conditions. Alternatively, and more preferably the method is performed by an informed user at home or in other words, without any medical supervision when the method is non-therapeutic.
The composition and method of the invention is useful to whiten teeth. This means that the composition is useful to preserve the white appearance of teeth or to restore the appearance periodically. Alternatively, the composition and the method is useful to lighten the teeth which are discoloured due to extrinsic or intrinsic stains (e.g., tobacco, coffee, tea and/or food or wine stains), fluorosis, developmental disturbances, bacteria, genetics, tetracycline antibiotics, trauma, blood decomposition, pigments present during development of teeth.
In one aspect the method is non-therapeutic. Preferably such a method is a cosmetic method. A therapeutic treatment starts from a pathological state, whereas a non-therapeutic treatment starts from a normal, healthy state. Application of such a method to a person is to improve his bodily appearance (cosmetic treatment).
In another aspect the method is therapeutic in nature. Treatment by therapy is defined as any treatment which is designed to cure, alleviate, remove or lessen the symptoms of, or prevent or reduce the possibility of contracting any disorder or malfunction of the human body, in this case it is teeth.

Use in accordance with the Invention

In accordance with yet another aspect is disclosed an aqueous gel composition of the first aspect for whitening teeth.
In one aspect the use is for non-therapeutic purpose. Alternatively, the use is for therapeutic purpose. The part of the description related to the method of the invention is also applicable *mutatis mutandis* to this aspect of the invention.
The following examples will more fully illustrate the embodiments of this invention. All parts, percentages and proportions referred to herein and in the appended claims are by weight unless otherwise illustrated.

## Examples

Example 1: WIO and ΔL* of compositions inside and outside the invention

The experiments were performed on Hydroxyapatite (HAP) discs because they are most suited for this purpose. In the first step the discs were stained with a staining solution (tea) by incubating the discs in an aqueous solution of tea for four days. All the discs turned dark brown in colour.

Absorbance measurement over the entire visible color spectrum were obtained using the CIELAB color scale (Commission International de L'Eclairage, 1978 and 1986) with a SpectroShade® Micro spectrophotometer (MHT, Italy). This scale quantifies color according to three parameters, L* (lightness-darkness); a* (red-green); and b* (yellow-blue) with increasing L* indicating a more aesthetically pleasing and desirable colour (whiter teeth).

Thereafter, the composition selected for the experiment was applied (70 to 80 mg per disc). The discs were then exposed to actinic light (Philips® LED lamp, 14 W B22) for 30 minutes. Distance between the HAP plate and the lamp was maintained at 5 cm.

After this step, the discs were washed with water and brushed using a standard calcium-based opaque toothpaste. The discs were then allowed to dry thoroughly for about 20 to 24 hours.

This was termed as the first cycle of treatment after which the absorbance measurements were repeated to determine the change in L* value so that difference between the initial L* value and the corresponding value after the first cycle of treatment could be determined.

After recording the L* value, the series of steps starting from (re) application of the selected composition and ending with the measurement of L*, were repeated. This was done until there was data pertaining to four cycles of treatment.

Note: All the compositions which were prepared for testing, contained water in addition to the photosensitizer and the gelling agent. In each composition, water formed the balance of the composition up to 100 wt%.

Table 1 shows the various compositions that were tested.

Table 2 shows the difference(s) in the L* values (ΔL*) at the end of each cycle vis-à-vis the corresponding values recorded immediately after staining the HAP discs (before the first cycle). The WIO was measured only at the end of the fourth cycle therefore it reflects the difference between the initial Whiteness Index of the stained HAP discs and the final Whiteness Index.

Table 1

| Reference number | Photosensitizer | Wt% | Gelling agent | Wt% |
|---|---|---|---|---|
| Control | -- | -- | Aristoflex® AVC | 1.0 |
| Comparative-1 | Rose Bengal | 0.02 | -- | - |
| Comparative-2 | Phloxine-B | 0.02 | -- | -- |
| Comparative-3 | Erythrosine-B | 0.02 | -- | -- |
| Inv-1 | Rose Bengal | 0.02 | Aristoflex® AVC | 1.0 |
| Inv-2 | Phloxine-B | 0.02 | Aristoflex® AVC | 1.0 |
| Inv-3 | Erythrosine-B | 0.02 | Aristoflex® AVC | 1.0 |

The ΔL* values observed at the end of each cycle and whiteness (WIO) at the end of the fourth cycle are shown in Table 2 below.

**Table 2**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control | 1 | 2 | 3 | 4 | -20 |
| Comparative-1 | 5 | 8 | 8 | 7 | -3 |
| Comparative-2 | 5 | 10 | 10 | 9 | -20 |
| Comparative-3 | 2 | 5 | 7 | 8 | -10 |

(continued)

| Reference number | Cycle 1 | Cycle 2 | Cycle 3 | Cycle 4 | |
|---|---|---|---|---|---|
| | ΔL* | ΔL* | ΔL* | ΔL* | WIO |
| Inv-1 | 5 | 15 | 24 | 40 | 60 |
| Inv-2 | 7 | 14 | 30 | 44 | 68 |
| Inv-3 | 7 | 15 | 25 | 42 | 60 |

The data in Table 2 indicates clearly that a composition which is devoid of either one of the polymer or the photosensitizer is not efficacious at all. The technical effects of progressive increase in L* cycle-by-cycle (as indicated by the ΔL* values) clearly points towards the inference that the photosensitizer and the gelling agent both must be present for effective whitening.

Example 2: WIO and ΔL* of compositions (The Effect of Amount of the Gelling Agent)

The compositions of Table 3 were prepared and tested as per details mentioned under Example 1. The corresponding data is summarized in Table 4.

**Table 3**

| Reference number | Photosensitizer | Wt% | Gelling agent | Wt% |
|---|---|---|---|---|
| Control | -- | -- | Aristoflex® AVC | 1.0 |
| Inv-4 | Rose Bengal | 0.02 | Aristoflex® AVC | 0.1 |
| Inv-5 | Rose Bengal | 0.02 | Aristoflex® AVC | 0.25 |
| Inv-6 | Rose Bengal | 0.02 | Aristoflex® AVC | 0.5 |
| Inv-7 | Rose Bengal | 0.02 | Aristoflex® AVC | 1.0 |
| Inv-8 | Rose Bengal | 0.02 | Aristoflex® AVC | 1.5 |
| Inv-9 | Rose Bengal | 0.02 | Aristoflex® AVC | 2.0 |

**Table 4**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control | 2 | 5 | 7 | 14 | -28 |
| Inv-4 | 8 | 12 | 7 | 15 | 15 |
| Inv-5 | 8 | 15 | 20 | 22 | 18 |
| Inv-6 | 6 | 12 | 18 | 35 | 48 |
| Inv-7 | 5 | 18 | 18 | 36 | 55 |
| Inv-8 | 10 | 24 | 32 | 38 | 58 |
| Inv-9 | 10 | 20 | 24 | 38 | 56 |

The data in Table 4 clearly indicates that all the compositions of the invention are efficacious and the efficacy increases with an increase in the wt% of the gelling agent but close to about 2 wt% the effect tapers off when the gelling agent is Aristoflex® AVC.

Example 3: WIO and ΔL* of compositions (The Effect of Various Cellulosic gelling agents)

The compositions of Table 5 were prepared and tested as per details mentioned under Example 1. The corresponding data is summarized in Table 6.

**Table 5**

| Reference number | Photosensitizer | Wt% | Gelling agent | Wt% |
|---|---|---|---|---|
| Control | -- | -- | | 1.0 |
| Inv-10 | Rose Bengal | 0.02 | Cellulose | 1.0 |
| Inv-11 | Rose Bengal | 0.02 | Hydroxyethyl cellulose | 1.0 |
| Inv-12 | Rose Bengal | 0.02 | Carboxymethyl cellulose | 1.0 |
| Inv-13 | Rose Bengal | 0.02 | Methyl cellulose | 1.0 |
| Inv-14 | Rose Bengal | 0.02 | Methocel® methylcellulose and hydroxypropyl methylcellulose polymers | 1.0 |
| Inv-15 | Rose Bengal | 0.02 | Hydroxypropylmethyl cellulose (Ashland K100M) | 1.0 |
| Inv-16 | Rose Bengal | 0.02 | Hydroxypropylmethyl cellulose (Ashland K35M) | 1.0 |

**Table 6**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control | 2 | 4 | 5 | 8 | -20 |
| Inv-10 | 10 | 15 | 20 | 24 | 18 |
| Inv-11 | 18 | 35 | 35 | 41 | 60 |
| Inv-12 | 18 | 20 | 24 | 28 | 20 |
| Inv-13 | 15 | 18 | 28 | 30 | 38 |
| Inv-14 | 18 | 20 | 28 | 36 | 58 |
| Inv-15 | 12 | 15 | 28 | 40 | 56 |
| Inv-16 | 15 | 18 | 25 | 30 | 25 |

The data in Table 6 indicates clearly that cellulose and all derivatives thereof (as tested) are highly efficacious.

Example 4: WIO and ΔL* of compositions (Effect of other gelling agents)

The compositions of Table 7 were prepared and tested in the same manner as per details mentioned under Example 1. The corresponding data is summarized in Table 8.

**Table 7**

| Reference number | Photosensitizer | Wt% | Gelling agent | Wt% |
|---|---|---|---|---|
| Control | -- | -- | | 1.0 |
| Comparative-4 | Rose Bengal | 0.02 | Xanthan gum | 1.0 |
| Inv-17 | Rose Bengal | 0.02 | Aristoflex® AVC | 1.0 |
| Inv-18 | Rose Bengal | 0.02 | Hydroxyethyl cellulose | 1.0 |
| Inv-19 | Rose Bengal | 0.02 | Carbopol® 940 | 1.0 |

**Table 8**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control | 2 | 4 | 5 | 8 | -20 |
| Comparative-4 | 5 | 12 | 10 | 12 | -10 |
| Inv-17 | 10 | 20 | 28 | 45 | 60 |
| Inv-18 | 25 | 30 | 35 | 48 | 50 |
| Inv-19 | 20 | 30 | 38 | 48 | 48 |

The data in Table 8 indicates clearly Aristoflex® AVC (from Clariant, INCI: Ammonium Acryloyldimethyltaurate/ vinyl pyrrolidone Copolymer), Hydroxyethyl cellulose and Carbopol® 940 (from, Lubrizol INCI Name: Carbomer) are highly efficacious but xanthan gum is not, as indicated by ΔL* and the WIO values.

Example 5: WIO and ΔL* of compositions (The Effect of other gelling agents)

The compositions of Table 9 were prepared and tested as per details mentioned under Example 1. The corresponding data is summarized in Table 10.

**Table 9**

| Reference number | Photosensitizer | Wt% | Gelling agent | Wt% |
|---|---|---|---|---|
| Control | -- | -- | | 2.0 |
| Comparative-5 | Rose Bengal | 0.02 | Xanthan Gum | 2.0 |
| Comparative-6 | Rose Bengal | 0.02 | Guar Gum | 2.0 |
| Comparative-7 | Rose Bengal | 0.02 | Locust Bean Gum | 2.0 |
| Comparative-8 | Rose Bengal | 0.02 | Carrageenan Gum | 2.0 |
| Comparative-9 | Rose Bengal | 0.02 | Acacia Gum | 2.0 |
| Comparative-10 | Rose Bengal | 0.02 | Chitosan | 2.0 |
| Inv-20 | Rose Bengal | 0.02 | Poly acrylic acid | 2.0 |
| Inv-21 | Rose Bengal | 0.02 | Hydroxyethyl cellulose 100000 to 250000 Dalton | 1.0 |
| Inv-22 | Rose Bengal | 0.02 | Hydroxyethyl cellulose | 2.0 |
| Inv-23 | Rose Bengal | 0.02 | Polyvinyl alcohol | 2.0 |
| Inv-24 | Rose Bengal | 0.02 | Gelatin | 2.0 |

**Table 10**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control | 2 | 4 | 5 | 8 | -20 |
| Comparative-5 | -2 | 2 | 6 | 8 | -12 |
| Comparative-6 | 2 | -8 | 2 | 2 | -20 |
| Comparative-7 | 2 | -5 | -10 | 0 | -42 |
| Comparative-8 | -2 | -8 | 8 | 0 | -2 |
| Comparative-9 | 4 | 4 | 2 | 8 | -16 |

(continued)

| Reference number | Cycle 1 | Cycle 2 | Cycle 3 | Cycle 4 | |
|---|---|---|---|---|---|
| | ΔL* | ΔL* | ΔL* | ΔL* | WIO |
| Comparative-10 | 2 | 2 | 8 | 18 | 8 |
| Inv-20 | 2 | 18 | 22 | 26 | 18 |
| Inv-21 | 15 | 24 | 26 | 38 | 50 |
| Inv-22 | 12 | 26 | 29 | 40 | 60 |
| Inv-23 | 8 | 18 | 24 | 34 | 45 |
| Inv-24 | 2 | 18 | 22 | 28 | 28 |

The data in Table 10 clearly indicates that compositions which contain xanthan gum, guar gum, locust bean gum, carrageenan, acacia gum and chitosan have little or no effect on ΔL* and WIO values. However, all the other compositions within the invention are highly efficacious.

Example 6: WIO and ΔL* of compositions (effect of sensitizers)

The following compositions of Table 11 were prepared and tested as per details mentioned under Example 1. The corresponding data is summarized in Table 12.

**Table 11**

| Reference number | Photosensitizer | Wt% | Polymer | Wt% |
|---|---|---|---|---|
| Control* | -- | -- | Aristoflex® AVC | 1.0 |
| Comparative-11 | Riboflavin | 0.02 | Aristoflex® AVC | 1.0 |
| Comparative-12 | Lumichrome | 0.02 | Aristoflex® AVC | 1.0 |
| Comparative-13 | Methylene Blue | 0.02 | Aristoflex® AVC | 1.0 |
| Inv-25 | Rose Bengal | 0.02 | Aristoflex® AVC | 1.0 |
| Inv-26 | Eosin Y | 0.02 | Aristoflex® AVC | 1.0 |
| Inv-27 | Erythrosine | 0.02 | Aristoflex® AVC | 1.0 |
| Inv-28 | Phloxine | 0.02 | Aristoflex® AVC | 1.0 |
| *The control composition contained 0.1 wt% hydrogen peroxide | | | | |

**Table 12**

| Reference number | Cycle 1 | Cycle 2 | Cycle 3 | Cycle 4 | |
|---|---|---|---|---|---|
| | ΔL* | ΔL* | ΔL* | ΔL* | WIO |
| Control* | 2 | 4 | 5 | 8 | -10 |
| Comparative-11 | 2 | 5 | 10 | 12 | -10 |
| Comparative-12 | -3 | 5 | 10 | 22 | 12 |
| Comparative-13 | -5 | -8 | -2 | 10 | 8 |
| Inv-25 | 5 | 15 | 18 | 30 | 68 |
| Inv-26 | 2 | 15 | 28 | 35 | 62 |
| Inv-27 | 5 | 12 | 25 | 30 | 40 |
| Inv-28 | 5 | 15 | 24 | 30 | 52 |

The data in Table 12 clearly indicates that compositions which contain other sensitizers (not of the xanthene class) are not efficacious. The technical effect is seen only in the case of the compositions according to the invention (Inv 25 to 28). Further, it is to be noted that the composition (Control*) is not as effective even inspite of the presence of the peroxide (oxidising agent).

Example 7: WIO and ΔL* of compositions (effect of organic solvents)

The compositions of Table 13 were prepared and tested as per details mentioned under Example 1. The corresponding data is summarized in Table 14.

**Table 13**

| Reference number | Photosensitizer | Wt% | Glycerine/ wt% | Gelling agent |
|---|---|---|---|---|
| Comparative-14 | Rose Bengal | 0.02 | -- | Aristoflex® AVC |
| Inv-29 | Rose Bengal | 0.02 | 10 | Aristoflex® AVC |
| Comparative-15 | Rose Bengal | 0.02 | 15 | Aristoflex® AVC |
| Comparative-16 | Rose Bengal | 0.02 | 17 | Aristoflex® AVC |

**Table 14**

| Reference number | Cycle 1 | Cycle 2 | Cycle 3 | Cycle 4 | |
|---|---|---|---|---|---|
| | ΔL* | ΔL* | ΔL* | ΔL* | WIO |
| Comparative-14 | 13 | 22 | 30 | 48 | 65 |
| Inv-29 | 11 | 18 | 24 | 41 | 48 |
| Comparative-15 | 4 | 10 | 22 | 32 | 15 |
| Comparative-16 | 15 | 12 | 22 | 34 | 20 |

The data in Table 14 indicates clearly how the addition of glycerine affects the photobleaching efficacy which is reflected as the Whiteness Index Observed. The WIO is the maximum when glycerine is at its minimum. The composition containing 10 % glycerine provides lower WIO as compared to the composition which has no glycerine but the reduction is not drastic. However, beyond 10% glycerine the WIO falls sharply.

## Claims

**1.** An aqueous gel composition for whitening teeth, comprising:

(i) at least one photosensitizer which is selected from a xanthene class of dye;
(ii) maximum of 10 wt% organic solvent;
(iii) less than 0.1 wt% oxidising agent; and,
(iv) a polymeric gelling agent other than those containing galactose, galactomannan or glucosamine units in their chemical structure.

**2.** A composition as claimed in claim 1 wherein said composition comprises 0.5 to 8 wt% organic solvent.

**3.** A composition as claimed in claim 1 or 2 wherein said organic solvent is at least one of glycerine, sorbitol, propyleneglycol, polyethyleneglycol or ethyleneglycol.

**4.** A composition as claimed in any of claims 1 to 3 wherein said photosensitiser is present in an amount of 0.001 to 5 wt%.

**5.** A composition as claimed in any of claims 1 to 3 wherein the xanthene class of dye is at least one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red,

Rhodamine 6G, Rhodamine B, Sulfonfluorescein, Tetrabromofluorescein Potassium Salt, Acid Red 91 (Eosin Bluish), Acid Red 94 (Rose Bengal), Acid Red 87(Eosin), Acid Red 92 (Phloxine B), 3,4,5,6-Tetrachlorofluorescein, Tetraiodofluorescein (Erythrosin B or Erythrosine B) and Phenylfluorone.

**6.** A composition as claimed in any of claim 5 wherein said photosensitizer is at least one of Rose Bengal, Eosin, Phloxine B or Erythrosine.

**7.** A composition as claimed in any of claims 1 to 6 wherein said polymeric gelling agent is at least one of a carbomer, a modified cellulose or a copolymer of ammonium acryloyldimethyltaurate and vinylpyrrolidone.

**8.** A composition as claimed in claim 7 wherein said modified cellulose is at least one of hydroxyethyl cellulose, sodium carboxymethyl cellulose, methyl cellulose or hydroxypropyl cellulose.

**9.** A kit-of-parts comprising:

(i) an aqueous gel composition as claimed in any of claims 1 to 8;
(ii) a source of actinic light;
(iii) optionally, an applicator for applying said composition to teeth; and,
(iv) further optionally, a set of instructions for use of said kit-of-parts.

**10.** A device for whitening teeth comprising:

(i) an aqueous gel composition as claimed in any of claims 1 to 8
(ii) an applicator configured to apply to the teeth said aqueous gel composition; and
(iii) a source of actinic light integral with said device.

**11.** A non-therapeutic method of whitening teeth comprising:

(i) providing an aqueous gel composition as claimed in any of claims 1 to 8;
(ii) applying said gel to teeth; and,
(iii) exposing said gel, so applied, to a source of actinic light.

**12.** Non-therapeutic use of an aqueous gel composition as claimed in any of claims 1 to 8 for whitening teeth.

**13.** A packaged product comprising an aqueous gel composition as claimed in any of claims 1 to 8.

**Patentansprüche**

**1.** Wässrige Gelzusammensetzung zum Weißen von Zähnen, umfassend:

(i) mindestens einen Photosensibilisator, der aus einer Xanthen-Farbstoffklasse ausgewählt ist;
(ii) maximal 10 Gew.-% organisches Lösungsmittel;
(iii) weniger als 0,1 Gew.-% Oxidationsmittel; und
(iv) ein polymeres Geliermittel, das von solchen verschieden ist, die Galactose-, Galactomannan- oder Glucosamin-Einheiten in ihrer chemischen Struktur enthalten.

**2.** Zusammensetzung wie in Anspruch 1 beansprucht, wobei die Zusammensetzung 0,5 bis 8 Gew.-% organisches Lösungsmittel umfasst.

**3.** Zusammensetzung wie in Anspruch 1 oder 2 beansprucht, wobei das organische Lösungsmittel mindestens eines von Glycerin, Sorbit, Propylenglycol, Polyethylenglycol oder Ethylenglycol ist.

**4.** Zusammensetzung wie in irgendeinem der Ansprüche 1 bis 3 beansprucht, wobei der Photosensibilisator in einer Menge von 0,001 bis 5 Gew.-% vorliegt.

**5.** Zusammensetzung wie in irgendeinem der Ansprüche 1 bis 3 beansprucht, wobei die Xanthen-Farbstoffklasse mindestens eine von Acid Red 52, Acid Red 289, Gallein, Bromopyrogallol Red, 2',7'-Dichlorfluoreszein-Natriumsalz,

9-(4'-Dimethylaminophenyl)-2,6,7-trihydroxyfluoronsulfathydrat, Dibromfluoreszein, Uranin K, Fluoreszein, Uranin, Pyrogallol Red, Rhodamin 6G, Rhodamin B, Sulfonfluoreszein, Tetrabromfluoreszein-Kaliumsalz, Acid Red 91 (Eosin Bluish), Acid Red 94 (Rose Bengal), Acid Red 87 (Eosin), Acid Red 92 (Phloxin B), 3,4,5,6-Tetrachlorfluoreszein, Tetraiodfluoreszein (Erythrosin B oder Erythrosine B) und Phenylfluoron ist.

**6.** Zusammensetzung wie in Anspruch 5 beansprucht, wobei der Photosensibilisator mindestens einer von Rose Bengal, Eosin, Phloxin B oder Erythrosin ist.

**7.** Zusammensetzung wie in irgendeinem der Ansprüche 1 bis 6 beansprucht, wobei das polymere Geliermittel mindestens eines von einem Carbomer, einer modifizierten Cellulose oder einem Copolymer von Ammoniumacryloyldimethyltaurat und Vinylpyrrolidon ist.

**8.** Zusammensetzung wie in Anspruch 7 beansprucht, wobei die modifizierte Cellulose mindestens eine von Hydroxyethylcellulose, Natriumcarboxymethylcellulose, Methylcellulose oder Hydroxypropylcellulose ist.

**9.** Kit von Teilen, umfassend:

(i) eine wässrige Gelzusammensetzung wie in irgendeinem der Ansprüche 1 bis 8 beansprucht;
(ii) eine aktinische Lichtquelle;
(iii) gegebenenfalls einen Applikator zum Aufbringen der Zusammensetzung auf Zähne; und
(iv) ferner gegebenenfalls einen Satz von Anweisungen zur Verwendung des Kits von Teilen.

**10.** Vorrichtung zum Weißen von Zähnen, umfassend:

(i) eine wässrige Gelzusammensetzung wie in irgendeinem der Ansprüche 1 bis 8 beansprucht;
(ii) einen Applikator, der konfiguriert ist zum Aufbringen der wässrigen Gelzusammensetzung auf die Zähne; und
(iii) eine aktinische Lichtquelle, die in die Vorrichtung integriert ist.

**11.** Nichttherapeutisches Verfahren zum Weißen von Zähnen, umfassend:

(i) Bereitstellen einer wässrigen Gelzusammensetzung wie in irgendeinem der Ansprüche 1 bis 8 beansprucht;
(ii) Aufbringen des Gels auf die Zähne; und
(iii) Bestrahlen des so aufgebrachten Gels mit einer aktinischen Lichtquelle.

**12.** Nichttherapeutische Verwendung einer wässrigen Gelzusammensetzung wie in irgendeinem der Ansprüche 1 bis 8 beansprucht für das Weißen von Zähnen.

**13.** Verpacktes Produkt, umfassend eine wässrige Gelzusammensetzung wie in irgendeinem der Ansprüche 1 bis 8 beansprucht.

**Revendications**

**1.** Composition de gel aqueuse pour le blanchiment des dents, comprenant :

(i) au moins un photosensibilisateur qui est choisi parmi un colorant de la classe du xanthène ;
(ii) au maximum 10 % en masse de solvant organique ;
(iii) moins de 0,1 % en masse d'agent oxydant ; et,
(iv) un agent gélifiant polymère différent de ceux contenant des unités galactose, galactomannane ou glucosamine dans leur structure chimique.

**2.** Composition selon la revendication 1, dans laquelle ladite composition comprend de 0,5 à 8 % en masse de solvant organique.

**3.** Composition selon la revendication 1 ou 2, dans laquelle ledit solvant organique est au moins un parmi la glycérine, le sorbitol, le propylèneglycol, le polyéthylèneglycol ou l'éthylèneglycol.

**4.** Composition selon l'une quelconque des revendications 1 à 3, dans laquelle ledit photosensibilisant est présent

dans une quantité de 0,001 à 5 % en masse.

**5.** Composition selon l'une quelconque des revendications 1 à 3, dans laquelle le colorant de la classe du xanthène est au moins un parmi rouge acide 52, rouge acide 289, galléine, rouge de bromopyrogallol, sel de sodium de 2',7'-dichlorofluorescéine, hydrate de sulfate de 9-(4'-diméthylaminophényl)-2,6,7-trihydroxyfluorone, dibromofluorescéine, uranine K, fluorescéine, uranine, rouge de pyrogallol, rhodamine 6G, rhodamine B, sulfonfluorescéine, sel de potassium de tétrabromofluorescéine, rouge acide 91 (éosine bleuâtre), rouge acide 94 (rose bengale), rouge acide 87 (éosine), rouge acide 92 (phloxine B), 3,4,5,6-tétrachlorofluorescéine, tétraiodofluorescéine (érythrosin B ou érythrosine B) et phénylfluorone.

**6.** Composition selon la revendication 5, dans laquelle ledit photosensibilisateur est au moins un de rose bengale, éosine, phloxine B ou érythrosine.

**7.** Composition selon l'une quelconque des revendications 1 à 6, dans laquelle ledit agent gélifiant polymère est au moins un d'un carbomère, une cellulose modifiée ou un copolymère d'acryloyldiméthyltaurate d'ammonium et vinylpyrrolidone.

**8.** Composition selon la revendication 7, dans laquelle ladite cellulose modifiée est au moins une de hydroxyéthylcellulose, sodium carboxyméthylcellulose, méthylcellulose ou hydroxypropylcellulose.

**9.** Kit d'éléments comprenant :

(i) une composition de gel aqueuse selon l'une quelconque des revendications 1 à 8 ;
(ii) une source de lumière actinique ;
(iii) éventuellement, un applicateur pour appliquer ladite composition aux dents ; et,
(iv) de plus éventuellement, un jeu d'instructions pour une utilisation dudit kit d'éléments.

**10.** Dispositif pour le blanchiment des dents comprenant :

(i) une composition de gel aqueuse selon l'une quelconque des revendications 1 à 8 ;
(ii) un applicateur configuré pour appliquer aux dents ladite composition de gel aqueuse ; et,
(iii) une source de lumière actinique d'une pièce avec ledit dispositif.

**11.** Procédé non-thérapeutique de blanchiment des dents comprenant :

(i) la fourniture d'une composition de gel aqueuse selon l'une quelconque des revendications 1 à 8 ;
(ii) l'application dudit gel aux dents ; et,
(iii) l'exposition dudit gel, ainsi appliqué, à une source de lumière actinique.

**12.** Utilisation non-thérapeutique d'une composition de gel aqueuse selon l'une quelconque des revendications 1 à 8 pour le blanchiment des dents.

**13.** Produit emballé comprenant une composition de gel aqueuse selon l'une quelconque des revendications 1 à 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1935395 A1
- US 2002122776 A
- WO 0018366 A1
- US 2003194383 AA
- WO 2011084746 A1
- WO 11084744 A1
- US 2009238778 AA
- US 80260660 A1
- EP 2338465 A1
- US 2006141422 A
- US 2014105832 AA
- WO 9940870 A1
- US 20030194383 A


### Non-patent literature cited in the description


- **GARCIA JA ; RUBINO M ; ROMERO J ; HITA E.** Measuring The Whiteness Of Human Teeth. *Color Research and Application,* 1993, vol. 18, 349-52